# EUROPEAN PATENT APPLICATION

(11) **EP 4 607 523 A1**
(43) Date of publication of application: **27.08.2025**
(21) Application number: 25157724.3
(22) Date of filing: 13.02.2025
(51) Int. Cl.: G16H 10/60, G16H 30/20, G16H 30/40, A61B 1/227, A61B 1/00, A61B 3/10

(54) **MEDICAL IMAGING DEVICE**

(30) Priority: 20.02.2024 US 202463555491 P
(71) Applicant: Welch Allyn, Inc., Skaneateles Falls, NY 13153 (US)
(72) Inventor: GUO, Lei, Skaneateles Falls, 13153 (US); ENDRES, Danielle R., Skaneateles Falls, 13153 (US); HESS, Joshua, Skaneateles Falls, 13153 (US); PERKINS, David G., Skaneateles Falls, 13153 (US); VILLARREAL, David, Skaneateles Falls, 13153 (US)
(74) Representative: Reddie & Grose LLP

(57) **Abstract**

An imaging device for capturing images through an eyepiece of an optical viewing device. The imaging device includes a camera that aligns with the eyepiece for capturing the images viewed through the eyepiece, and a display for displaying the images captured by the camera. The imaging device determines a type of the optical viewing device. The imaging device presents a workflow on the display based on the type of the optical viewing device. The imaging device captures metrics related to user interactions with the workflow presented on the display and the images captured by the camera during the workflow.

## Description

### BACKGROUND

Optical viewing devices are used for examining patients as part of routine examinations. Examples of optical viewing devices can include, without limitation, an otoscope for assessing the ears of a patient, an ophthalmoscope for assessing the eyes of a patient, and a dermatoscope for assessing the skin of a patient. Different types of examinations and workflows are typically performed based on the type of optical viewing device being used.

### SUMMARY

In general terms, the present disclosure relates to imaging for optical viewing devices. In one possible configuration, an imaging device is configured for use on optical viewing devices, and the imaging device tracks usage and image quality metrics from examinations performed using the optical viewing devices. Various aspects are described in this disclosure, which include, but are not limited to, the following aspects.

One aspect relates to an imaging device for capturing images through an eyepiece of an optical viewing device, the imaging device comprising: a camera configured for alignment with the eyepiece of the optical viewing device for capturing the images viewed through the eyepiece; a display for displaying the images captured by the camera; at least one processing device; and at least one computer readable data storage device storing software instructions that, when executed by the at least one processing device, cause the imaging device to: determine a type of the optical viewing device; present a workflow on the display based on the type of the optical viewing device; and capture metrics related to user interactions with the workflow presented on the display and the images captured by the camera during the workflow.

Another aspect relates to a method of capturing images through an eyepiece of an optical viewing device, the method comprising: providing an imaging device for attachment to the optical viewing device; presenting a workflow on the imaging device for capturing the images based on a type of optical viewing device, the workflow including tools for annotating the images on a display; capturing metrics related to user interactions with the workflow, and quality characteristics of the images captured during the workflow; and generating a recommendation based on the metrics.

Another aspect relates to a system for imaging an anatomy, the system comprising: an optical viewing device including an instrument head having an eyepiece; and an imaging device attached to the optical viewing device, the imaging device including: a camera configured for alignment with the eyepiece of the instrument head for capturing the images viewed through the eyepiece; a display for displaying the images captured by the camera; at least one processing device; and at least one computer readable data storage device storing software instructions that, when executed by the at least one processing device, cause the device to: determine a type of the optical viewing device; present a workflow on the display based on the type of the optical viewing device; and capture metrics related to user interactions with the workflow presented on the display and the images captured by the camera during the workflow.

The invention will now be further described by way of example with reference to the accompanying drawings, in which:
FIG. 1 shows examples of different types of optical viewing devices, each optical viewing device is shown attached to an imaging device.
FIG. 2 is an isometric view of an example of an optical viewing device shown in FIG. 1, the optical viewing device shown from a physician perspective.
FIG. 3 is another isometric view of the optical viewing device of FIG. 2, the optical viewing device shown from a patient perspective.
FIG. 4 is an isometric view of an example of the imaging device attached to the optical viewing device of FIG. 2, the imaging device shown from the physician perspective.
FIG. 5 is a front isometric view of the imaging device of FIG. 4.
FIG. 6A is a front view of the imaging device of FIG. 4.
FIG. 6B is a rear view of the imaging device of FIG. 4.
FIG. 6C is a top view of the imaging device of FIG. 4.
FIG. 7 is an isometric view showing a camera of the imaging device of FIG. 4.
FIG. 8 schematically illustrates an example of a method of improving security of protected health information on the imaging device of FIGS. 1-7.
FIG. 9 illustrates an example of a graphical user interface that can be used in operations of the method of FIG. 8 to request and receive user credentials.
FIG. 10 illustrates an example of a graphical user interface for searching for a patient in accordance with an example of a workflow presented in the method of FIG. 8.
FIG. 11 illustrates an example of a graphical user interface optimized for capturing images and/or videos through an eyepiece of a type of optical viewing device in accordance within an example of the workflow presented in the method of FIG. 8.
FIG. 12 illustrates an example of a graphical user interface for saving an examination in accordance with an example of the workflow presented in the method of FIG. 8.
FIG. 13 illustrates an example of a graphical user interface displayed in the workflow of the method of FIG. 8 after a user selects a save icon in the graphical user interface of FIG. 12.
FIG. 14 illustrates an example of an export screen that can be generated in an operation of the method of FIG. 8.
FIG. 15 schematically illustrates an example of an examination report generated in an operation of the method of FIG. 8.
FIG. 16 illustrates an example of a graphical user interface that can be displayed in an operation of the method of FIG. 8, the graphical user interface includes a training window that has a play icon that is selectable to playback a video that provides tips and techniques to improve user competency of the imaging device and/or the optical viewing device of FIGS. 1-7.
FIG. 17 illustrates another example of a graphical user interface that can be displayed in an operation of the method of FIG. 8, the graphical user interface includes a training window that has a play icon that is selectable to playback a video that explains how to upload exams to a secure destination such as by using the export screen of FIG. 14.
FIG. 18 schematically illustrates an example of a usage report that can be generated by a user analytics system communicatively connected to the imaging device of FIGS. 1-7.
FIG. 19 illustrates an example of a graphical user interface that can be displayed on the imaging device of FIGS. 1-7.
FIG. 20 illustrates an exemplary architecture of a computing device of the imaging device shown in any of the above figures.

### DETAILED DESCRIPTION

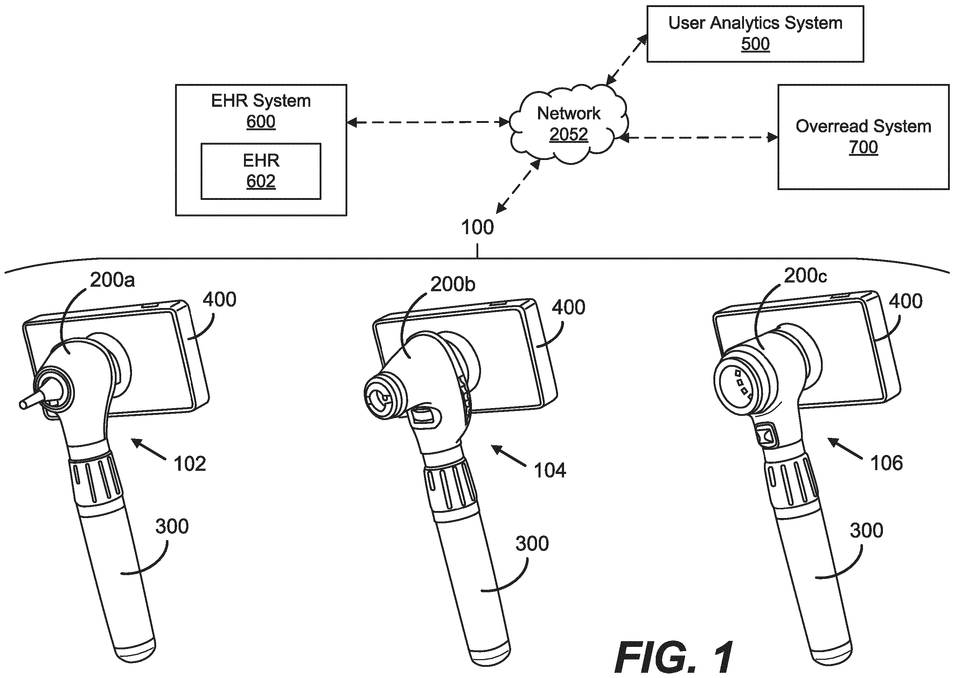

FIG. 1 shows examples of different types of optical viewing devices 100. The optical viewing devices 100 include a first type of optical viewing device 102, a second type of optical viewing device 104, and a third type of optical viewing device 106. In the example illustrated in FIG. 1, the first type of optical viewing device 102 is an otoscope, the second type of optical viewing device 104 is an ophthalmoscope, and the third type of optical viewing device 106 is a dermatoscope. Additional types of the optical viewing devices 100 can exist, and the disclosure provided herein is not limited to otoscopes, ophthalmoscopes, and dermatoscopes.

As shown in FIG. 1, each type of optical viewing device 100 includes an instrument head 200 attached to an instrument handle 300. The instrument head 200 can include a light source and optics for viewing an anatomical area of interest through an eyepiece.

The instrument handle 300 can include a power source that powers the light source and other components of the instrument head 200. For example, the instrument handle 300 can include rechargeable batteries, disposable batteries, or a tether to a wall transformer for supplying electrical power to the components of the instrument head 200.

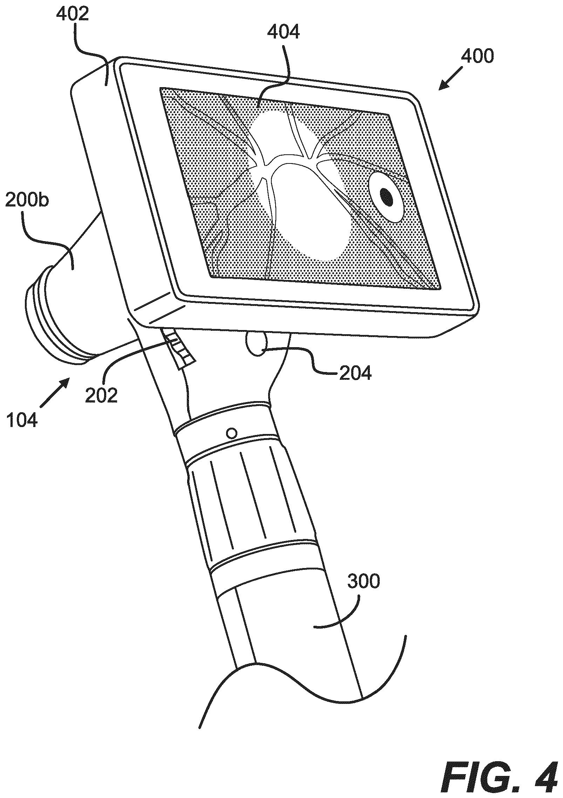

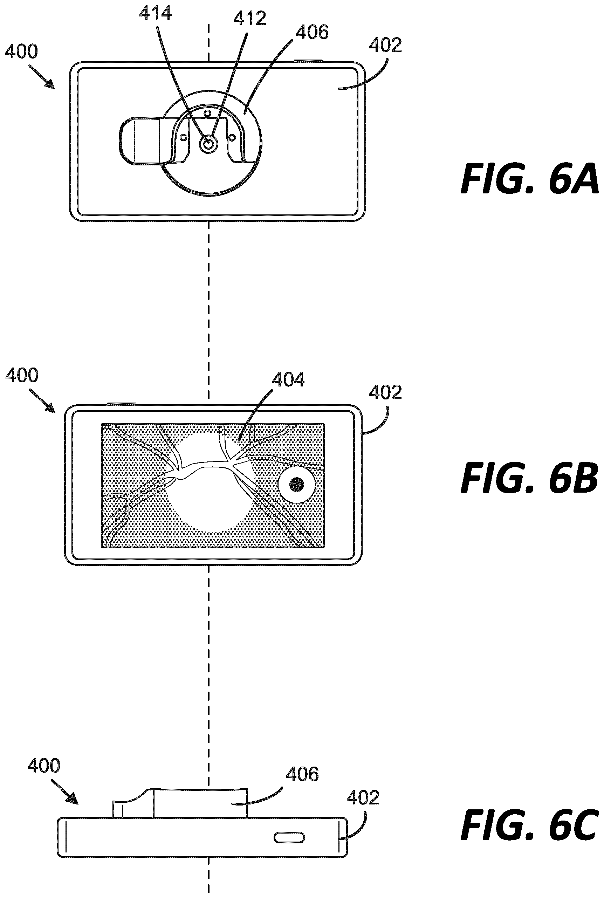

As shown in FIG. 1, the instrument head 200 of each type of optical viewing device 100 includes an imaging device 400 attached thereto. The imaging devices 400 are configured to capture images and videos through an eyepiece of the instrument head 200. The images and videos are displayed on a display 404 mounted on a rear surface of the imaging device 400 (see FIGS. 4 and 6B) for viewing by a physician. The imaging device 400 can include aspects described in U.S. Provisional Patent Application No. 63/503,219, entitled Imaging for Optical Viewing Devices, filed May 19, 2023, U.S. Provisional Patent Application No. 63/505,771, entitled Image Capture for Optical Viewing Devices, filed June 2, 2023, and U.S. Provisional Patent Application No. 63/514,250, entitled Image Streaming for Optical Viewing Device, filed July 18, 2023, which are herein incorporated by reference in their entireties.

As shown in FIG. 1, the imaging devices 400 are universal with respect to the optical viewing devices 100 such that an imaging device 400 can be interchangeably used on the instrument head 200a of the first type of optical viewing device 102, the instrument head 200b of the second type of optical viewing device 104, and the instrument head 200c of the third type of optical viewing device 106. The instrument handle 300 may also be universal with respect to the first type of optical viewing device 102, the second type of optical viewing device 104, and the third type of optical viewing device 106 such that the instrument handle 300 can be interchangeably used between the different types of optical viewing devices.

As shown in FIG. 1, the imaging devices 400 are communicatively connected over the network 2052 to a user analytics system 500. As will be described in more detail, the user analytics system 500 can analyze attributes related to the usage of the imaging devices 400 to prepare recommendations for implementation on the imaging devices 400 to improve user competency, and efficacy of the imaging devices 400 and/or the optical viewing devices 100.

Images, videos, and other data captured by the imaging devices 400 can be transferred over the network 2052 to an EHR system 600 for storage in an electronic health record (EHR) 602. As described herein, the terms electronic medical records (EMRs) and electronic patient record (EPRs) can be used interchangeably with EHRs. The EHR system 600 collects patient electronically stored health information in a digital format (e.g., EHRs 602). As such, the EHR system 600 maintains a plurality of EHRs 602 for a plurality of patients. Each EHR 602 can be shared across different health care settings. For example, the EHRs 602 are shared through network-connected, enterprise-wide information systems or other information networks and exchanges. The EHRs 602 may include a range of data, including demographics, medical history, medication and allergies, immunization status, laboratory test results, radiology images, vital signs, personal statistics like age and weight, and billing information.

Additionally, the images, videos, and other data captured by the imaging devices 400 can be transferred over a network 2052 to an overread system 700. The overread system 700 can provide the images, videos, and other data captured by the imaging devices 400 for review by trained medical specialists such as ear, nose, and throat (ENT) specialists, ophthalmologists, and dermatologists, or by trained technicians. Additionally, or alternatively, the overread system 700 can include algorithms, including artificial intelligence and machine learning algorithms, which can be used to analyze the images, videos, and data captured by the imaging devices 400.

The overread system 700 generates results from analysis of the images, videos, and other data captured by the imaging device 400. The overread system 700 can transfer the results to the imaging device 400 or to another device over the network 2052. Accordingly, two-way communications between the imaging device 400 and the overread system 700 can be provided over the network 2052. The overread system 700 can be remotely located with respect to the imaging device 400. In some examples, the overread system 700 includes a cloud server.

The network 2052 can include any type of wired or wireless connections, or any combinations thereof. Examples of wireless connections include cellular network connections such as 4G or 5G. The wireless connections can also be accomplished using Wi-Fi, ultra-wideband (UWB), Bluetooth, radio frequency identification (RFID), and the like.

The images, videos, and other data captured by the imaging devices 400 can be transferred over the network 2052 to the EHR system 600, the overread system 700, and/or the user analytics system 500 using the Fast Healthcare Interoperability Resources (FHIR) standard, which is a set of rules and specifications for exchanging electronic healthcare data in a wide range of settings and with different health care information systems. The FHIR standard describes data formats and elements (known as "resources") and an application programming interface (API) for exchanging the EHRs 602. Alternatively, the images, videos, and other data captured by the imaging devices 400 can be transferred over the network 2052 to the EHR system 600, the overread system 700, and/or the user analytics system 500 using the HL7 version 2.x and HL7 version 3.x data format standards for exchanging electronic healthcare data.

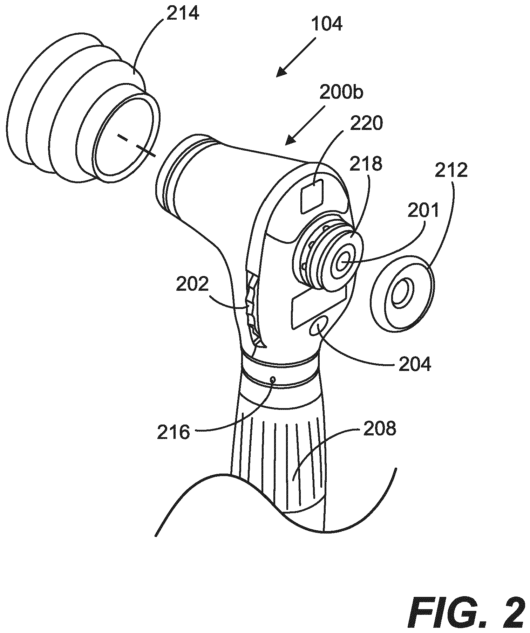

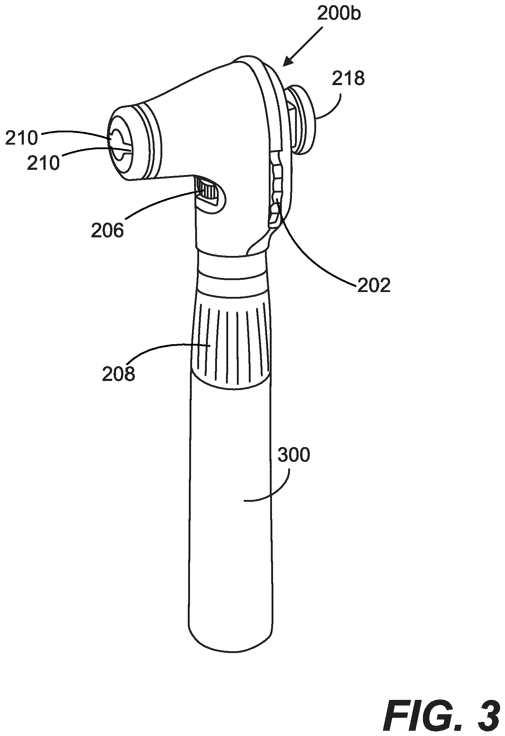

FIGS. 2 and 3 are isometric views of an example of the second type of optical viewing device 104. In FIG. 2, the second type of optical viewing device 104 is shown from a physician perspective. In FIG. 3, the second type of optical viewing device 104 is shown from a patient perspective. As discussed above, the second type of optical viewing device 104 is an example of an ophthalmoscope. While FIGS. 2 and 3 show the second type of optical viewing device 104, the first type of optical viewing device 102 and the third type of optical viewing device 106 can include similar components and features such that the following description can similarly apply to the first and third types of optical viewing device 102, 106.

Referring now to FIGS. 2 and 3, the second type of optical viewing device 104 includes a diopter focus wheel 202 and a diopter readout 204. The diopter focus wheel 202 can be used to adjust a focus of an eyepiece 201. The diopter focus wheel 202 can be used to correct the refractive errors of the user of the optical viewing device 104 and the patient. For example, the diopter focus wheel 202 can be used to provide a positive dioptric value to accommodate for hyperopia eyesight (farsightedness) of both the user of the optical viewing device 104 and the patient, and to provide a negative dioptric value to accommodate for myopia eyesight (nearsightedness) of both the user of the optical viewing device 104 and the patient. The dioptric values adjusted by using the diopter focus wheel 202 are displayed in the diopter readout 204.

The instrument head 200b of the second type of optical viewing device 104 can further include a filter wheel 206 to select a filter for viewing through the eyepiece 201. For example, the filter wheel 206 can be used to select a reticle target to measure the optic disc, a cobalt blue filter to detect corneal abrasions, a red-free filter, and additional types of filters.

The second type of optical viewing device 104 can further include a light control 208 for controlling illumination from the light source, disc alignment lights 210 (e.g., red for right eye exams; yellow for left eye exams), an eyepiece bumper 212, an optional patient eye cup 214, an optional locking collar 216, and an eyepiece housing 218. As will be described in more detail below, the imaging device 400 includes a bracket that removably attaches to the eyepiece housing 218 for securing the imaging device 400 to the instrument head 200b.

As further shown in FIG. 2, the second type of optical viewing device 104 can include an identifier 220. While the identifier 220 is described with reference to the second type of optical viewing device 104, the first and third types of optical viewing devices 102, 106, as well as additional types of optical viewing devices can similarly include the identifier 220, as described herein. As will be described in more detail, the identifier 220 provides machine-readable data that can be detected by the imaging device 400 to detect attachment of the imaging device 400 to the instrument head 200, and to convey additional information such as the type of the instrument head 200 (i.e., whether the instrument head 200 is for an otoscope, an ophthalmoscope, a dermatoscope, or another type of optical viewing device).

In some examples, the identifier 220 is a wireless antenna that transmits a wireless signal that is detected by the imaging device 400 when the imaging device 400 is attached to the instrument head 200. In some examples, the wireless signal transmitted by the identifier 220 to the imaging device 400 can include a radio frequency identification (RFID) signal, a near field communication (NFC) signal, a Bluetooth signal, a Wi-Fi signal, or other similar wireless signals. The identifier 220 can include a passive antenna or tag that is activated by an active antenna on the imaging device 400 to transmit the wireless signal when the imaging device 400 is in close proximity to the instrument head 200 such as when it is attached thereto.

In some further examples, the identifier 220 can provide additional types of machine-readable data that can be detected by the imaging device 400. For example, the identifier 220 can include a quick response (QR) code or other type of machine-readable label that can be read by a primary camera or a secondary camera of the imaging device 400.

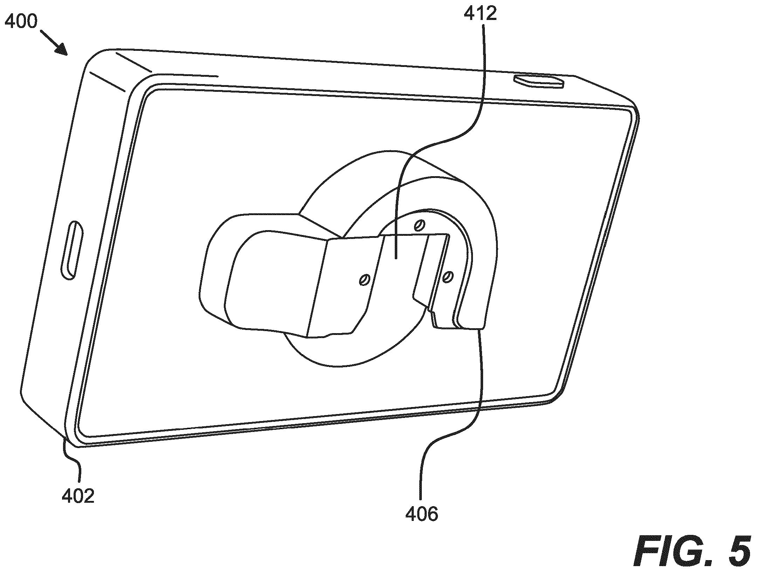

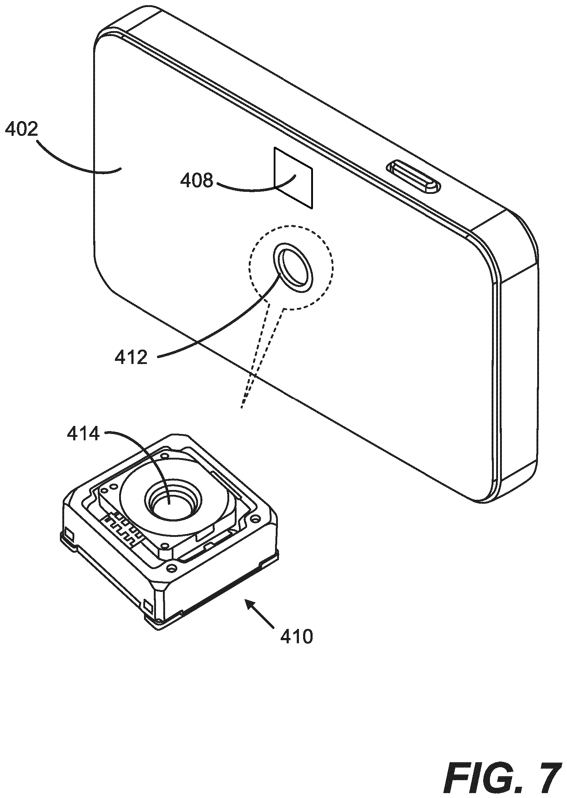

FIG. 4 is a rear isometric view of an example of the imaging device 400 attached to the second type of optical viewing device 104, shown from the physician perspective. FIG. 5 is a front isometric view of the imaging device 400. FIG. 6A is a front view of the imaging device 400. FIG. 6B is a rear view of the imaging device 400. FIG. 6C is a top view of the imaging device 400. FIG. 7 is an isometric view showing a camera 410 of the imaging device 400. Referring now to FIGS. 4-7, the imaging device 400 captures images viewed from the eyepiece 201 of the instrument head 200b of the second type of optical viewing device 104.

While FIGS. 4-6 show the imaging device 400 attached to the instrument head 200b of the second type of optical viewing device 104, the imaging device 400 can be similarly attached to the instrument heads 200a, 200c of the first type of optical viewing device 102 and the third type of optical viewing device 106 for capturing images and videos from the eyepieces of the first type of optical viewing device 102 and the third type of optical viewing device 106.

As shown in FIGS. 4-6, the imaging device 400 includes a housing 402. In this example, a bracket 406 is integrated with a back surface of the housing 402. The bracket 406 allows the imaging device 400 to physically attach to the instrument head 200b. For example, the bracket 406 can be fixed around the eyepiece housing 218 (see FIGS. 2 and 3) of the instrument head 200b. In alternative examples, the bracket 406 can be an accessory that attaches to the housing 402 for attachment of the imaging device 400 to the optical viewing devices 100.

As shown in FIGS. 5 and 7, the housing 402 further includes an aperture 412 for a lens 414 of the camera 410. The camera 410 is mounted inside the housing 402 of the imaging device 400. When the imaging device 400 is mounted to the instrument head 200b, the camera 410 is aligned with the eyepiece 201 of the instrument head 200b for capturing images viewed through the eyepiece 201 of the instrument head 200b. The camera 410 is centrally mounted inside the housing 402 to provide even balance and weight distribution for when the imaging device 400 is attached to the instrument head 200b, thereby improve the ergonomics of the assembly. A protrusion of the lens 414 beyond the back surface of the housing 402 is minimized such that the lens 414 is substantially flush with the back surface of the housing 402.

The camera 410 can include features such as auto focus, auto-exposure, auto white-balance, and image stabilization. The camera 410 can include a 12MP color image sensor. As an illustrative example, the camera 410 can include an equivalent focal length (on 35mm film) of 52-77mm, 4K (30FPS) video recording with 4000x3000 pixel resolution, and a record time of 90 minutes at 4K resolution, 1 minute per clip. Alternative camera parameters are possible.

The housing 402 is compact and lightweight. In some examples, the housing 402 includes a protective overmold having a base layer of plastic material, and a top layer of rubber to provide shock absorption and improved grip. The housing 402 can include one or more ports such as a USB-C port for charging the battery, and for data transferring including uploading images and videos captured by the camera 410 to another device. As an illustrative example, the housing 402 can have a thickness (e.g., distance between the lens 414 of the camera 410 and the display 404) that is less than 25 mm, and a weight that is less than 250g. The housing 402 can include a power button to turn on/off and wake up the imaging device 400. The housing 402 houses an integrated, rechargeable battery that can, for example, power 90 minutes of 4K video recording by the camera 410, and 3-4 hours of screen time on the display 404.

As shown in FIG. 7, the imaging device 400 can include a detector 408 that detects the machine-readable data from the identifier 220 on the instrument head 200 (see FIG. 2) to detect attachment of the imaging device 400 to the instrument head 200, and to detect additional information such as the type of the instrument head 200 (i.e., whether the instrument head 200 is for an otoscope, an ophthalmoscope, a dermatoscope, or another type of optical viewing device).

In some examples, the detector 408 is a wireless antenna that detects a wireless signal from the identifier 220 on the instrument head 200 when the imaging device 400 is attached to the instrument head 200. In some examples, the detector 408 on the imaging device 400 can detect a radio frequency identification (RFID) signal, a near field communication (NFC) signal, a Bluetooth signal, a Wi-Fi signal, or other similar wireless signals emitted from the instrument head 200. The detector 408 can include an active antenna or tag that activates a passive antenna or tag on the instrument head 200 to receive a transmission of the wireless signal. In some examples, the active antenna is mounted on the imaging device 400 in a location that corresponds to the placement of the passive antenna on the instrument head 200 such that the active antenna activates the passive antenna when in close proximity to the passive antenna such as when the imaging device 400 is attached to the instrument head 200.

In some further examples, the detector 408 can include a secondary camera that can read a quick response (QR) code or other type of machine-readable label placed on the instrument head 200. The secondary camera can read the machine-readable label to detect attachment of the imaging device 400 to the instrument head 200, as well as to determine the type of the instrument head 200 (i.e., whether the instrument head 200 is for an otoscope, an ophthalmoscope, a dermatoscope, or another type of optical viewing device). The secondary camera can be mounted on the imaging device 400 in a location that corresponds to the placement of the machine-readable label on the instrument head 200.

As further shown in FIGS. 4-7, the imaging device 400 includes the display 404 for displaying the images, videos, and other data captured by the imaging device 400. The display 404 can include a touchscreen that displays the images, the videos, and the other data, and that also receives inputs from a user. For example, the display 404 can be used by a user of the imaging device 400 to adjust the settings of the camera 410 (e.g., focus, exposure, white balance, FOV/zoom); tapping the display 404 to trigger focus and lock; adjust settings of the display 404 such as the screen brightness; provide a virtual keyboard to type in information; display a battery-life indicator; provide video recording controls (e.g., start, stop, save, delete, review, upload; provide a sliding bar to go through video frames, pinch-zoom to enlarge; display arrow(s) to indicate image orientation; and display one or more stamps (e.g., date, time, filter type, and the like) on saved images and videos.

The display 404 can include a true color multi-touch screen (in-plane switching (IPS), or light-emitting diode (LED)). The display 404 can have a bezel-less design (e.g., full-screen display). The display 404 can have a resolution of at least 250 pixels per inch (PPI), a diagonal screen size of about 2 inches to about 5 inches, an aspect ratio of 16:9/4:3, a maximum brightness of 500 nits. The display 404 can also include features such as screen auto off, and wake up by power button or tapping the display 404.

Additionally, the imaging device 400 can provide haptic feedback based on touches detected on the display 404, or selection of one or more physical push buttons on the housing 402 of the imaging device 400. The haptic feedback can include vibrations to give feedback rather than audible beeps to quickly communicate to the user actions such as when video capture has initiated or when a captured video is ready for playback.

Healthcare facilities such as hospitals, medical offices, clinics, and the like generally have secure protocols for handling, storing, and transferring medical data in view of regulations established under the Health Insurance Portability and Accountability Act (HIPAA) that address the use and disclosure of protected health information by covered entities. The images, videos, and other data captured by the imaging device 400 can include protected health information.

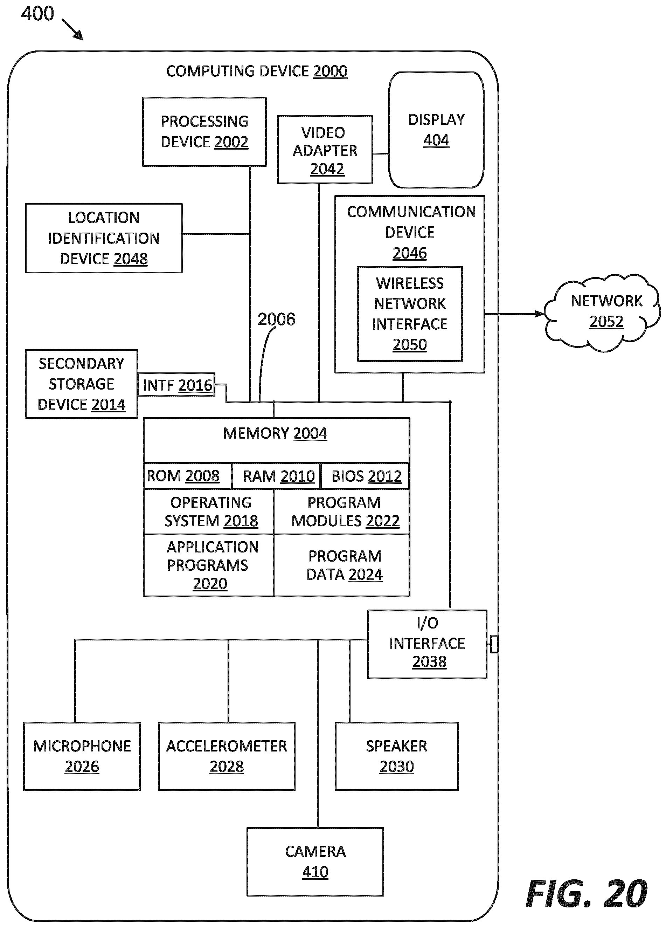

To maintain the confidentiality of protected health information captured on the imaging device 400, the images, videos, and other data captured by the imaging device 400 are deleted from the imaging device 400 after the images, videos, and other data are transferred to another device or system such as the EHR system 600 or the overread system 700. For example, the images, videos, and other data can be stored on a random-access memory (RAM) 2010 of the imaging device 400 (see FIG. 20) while the imaging device 400 is being used to capture images and/or videos during an examination performed using an optical viewing device 100 with the imaging device 400 attached thereto. When the examination is complete, a report is generated on the imaging device 400 using the images, videos, and other data stored on the random-access memory (RAM) 2010. After the report is transferred to another device or system such as the EHR system 600 or the overread system 700, the images, videos, and other data are deleted from the RAM 2010 to mitigate risk that such information is accessed by an unauthorized entity.

When an examination recorded by the imaging device 400 is incomplete or the report is not transferred to another device or system, the images, videos, and other data captured during the examination can remain on the RAM 2010 of the imaging device 400. This can lead to potential security vulnerabilities in which the images, videos, and other data can be accessed or stolen by an unauthorized user or entity. To mitigate such security vulnerabilities, it can be desirable to track usage and data transfer metrics from the imaging device 400 to improve the security of protected health information that may reside on the imaging device 400. Additionally, user competency and training for improving exam efficacy and image quality can be identified by tracking usage and data transfer metrics from the imaging device 400.

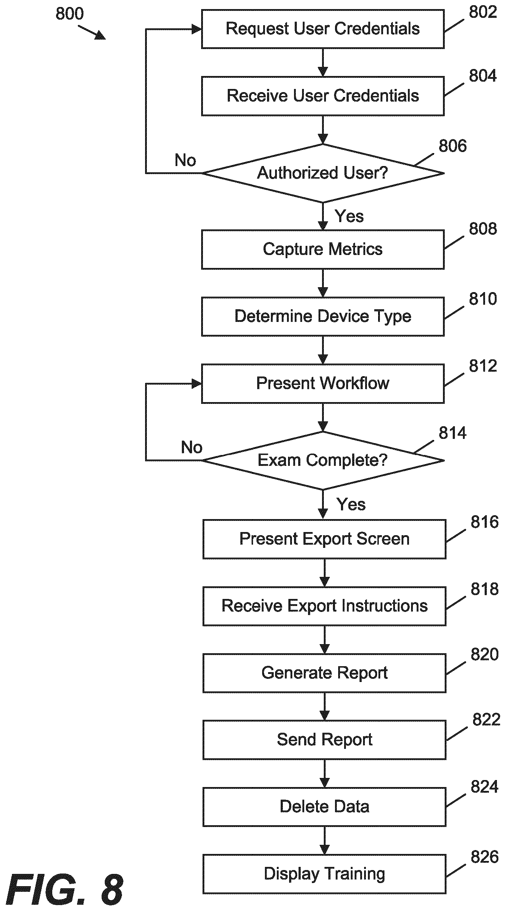

FIG. 8 schematically illustrates an example of a method 800 of improving security of protected health information on the imaging device 400. The method 800 can be implemented on the imaging device 400. In some examples, the method 800 is performed by execution of software instructions stored on a memory of the imaging device 400.

As shown in FIG. 8, the method 800 includes an operation 802 of requesting user credentials. As an illustrative example, when the imaging device 400 is powered on, the imaging device 400 displays a graphical user interface on the display 404 that requests a user of the imaging device 400 to enter their user credentials for authentication. The user credentials can include a username and password, a personal identification number (PIN), machine-readable data, biometric data, or some other type of data that can be used to authenticate the user.

The method 800 includes an operation 804 of receiving the user credentials. In some examples, the user credentials are received in operation 804 from a scan of a machine-readable label such as a barcode or QR code that identifies the user of the imaging device 400. For example, the user can use the camera 410 to scan a machine-readable label that is printed on a lanyard or other accessory possessed by the user. Alternatively, operation 804 can include receiving the user credentials from the user entering a username, password, and/or PIN. As further example, operation 804 can include receiving a scan of the user's face, fingerprint, or some other type of biometric data that can be used to authenticate the user. In some instances, two-factor authentication is performed where the user enters a code included in a notification, a text message, or an email sent to another device associated with the user such as a smartphone.

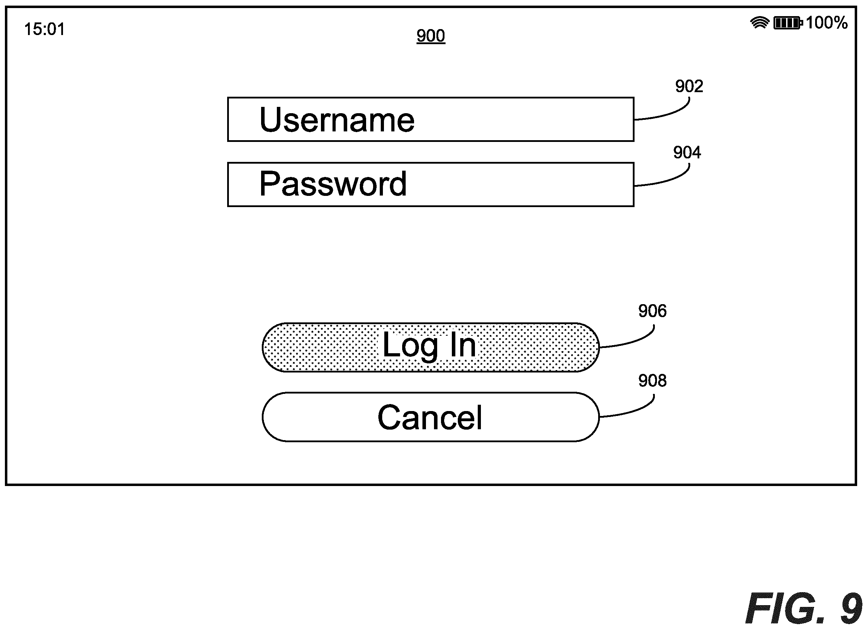

FIG. 9 illustrates an example of a graphical user interface 900 that can be used in operations 802, 804 of the method 800 to request and receive the user credentials. The graphical user interface 900 has a first field 902 for the user to enter their username, and a second field 904 for the user to enter their password. Once the user has entered the requested data in the first and second fields 902, 904, the user can select an input 906 to submit the username and password for authentication. Alternatively, the user can select an input 908 to cancel the request.

Referring back to FIG. 8, the method 800 includes an operation 806 of determining whether the user credentials received in operation 804 are valid for authenticating the user of the imaging device 400. When the user credentials are invalid (i.e., "No" in operation 806), such as when the username and/or password entered by the user are not recognized as belonging to an authorized user, the method 800 returns to operation 802 of requesting the user credentials. In this manner, only authorized users are allowed to use the imaging device 400.

In some examples, the imaging device 400 permits use of the device without requiring valid user credentials such as during a training or testing mode. In such training or testing mode, the imaging device 400 can be used to capture images, videos, and/or other types of data without associating the data with a real patient such that protected health information is not generated on the device. In some examples, the training or testing mode allows the images, videos, and/or other types of data to be saved on the RAM 2010 of the imaging device 400.

As shown in FIG. 8, when the user credentials are valid (i.e., "Yes" in operation 806), the method 800 proceeds to an operation 808 of capturing metrics during use of the imaging device 400. The metrics captured in operation 808 can be stored in the RAM 2010 of the imaging device 400. As will be described in more detail below, the metrics can be used to enhance security of protected health information on the imaging device 400, as well as improve user competency and training by identifying areas where the imaging device 400 and/or the optical viewing device 100 can be more effectively used to examine a patient.

The method 800 includes an operation 810 of determining a type of optical viewing device 100 that the imaging device 400 is attached to. Operation 810 can include determining the instrument head 200 belongs to the first type of optical viewing device 102 (e.g., an otoscope), the second type of optical viewing device 104 (e.g., an ophthalmoscope), the third type of optical viewing device 106 (e.g., a dermatoscope), or another type of optical viewing device.

Operation 810 can include determining the type of optical viewing device 100 based on a detection of the instrument head 200 such as by the detector 408 detecting the identifier 220 on the instrument head 200 or the camera 410 reading a machine-readable label on the instrument head 200. Alternatively, or additionally, operation 810 can include determining the type of optical viewing device 100 based on a confirmation received on the display 404 (in examples where the display 404 is a touch sensitive touchscreen). Examples of determining the type of optical viewing device 100 are described in U.S. Provisional Patent Application No. 63/607,600, entitled Workflows and Graphical User Interfaces for Optical Viewing Devices, filed December 8, 2023, and U.S. Provisional Patent Application No. 63/617,234, entitled Workflows and Graphical User Interfaces for Optical Viewing Devices, filed January 3, 2024, which are herein incorporated by reference in their entireties.

The metrics captured in operation 808 of the method 800 can include the type of optical viewing device 100 determined in operation 810 such as whether the imaging device 400 is attached to the first type of optical viewing device 102 (e.g., an otoscope), the second type of optical viewing device 104 (e.g., an ophthalmoscope), the third type of optical viewing device 106 (e.g., a dermatoscope), or another type of optical viewing device.

The method 800 includes an operation 812 of presenting a workflow on the display 404 of the imaging device 400 based on the type of optical viewing device 100 determined in operation 810. The workflow can include a series of graphical user interfaces that are displayed on the display 404 for recording an examination of a patient performed by using the type of optical viewing device 100 determined in operation 810.

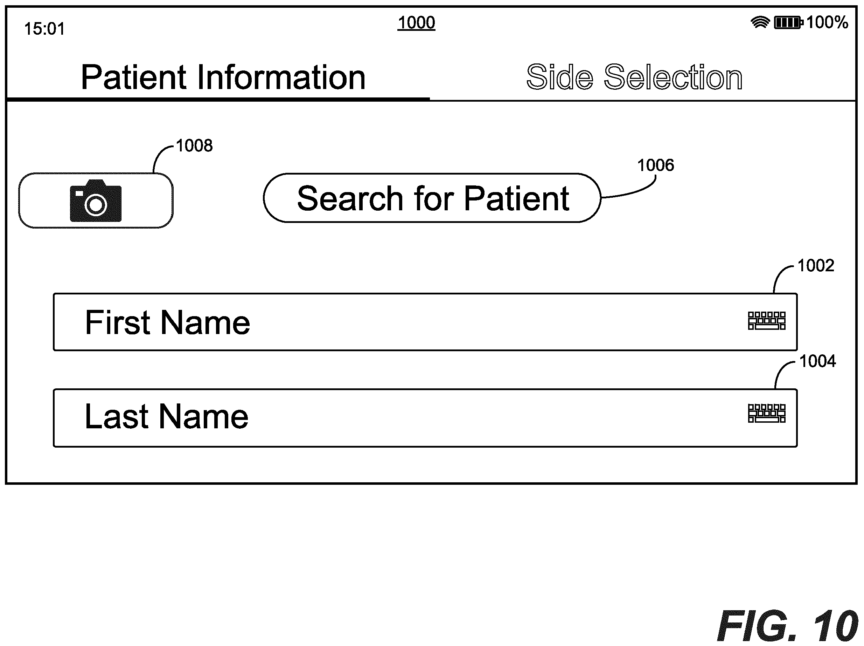

FIG. 10 illustrates an example of a graphical user interface 1000 that can be displayed in the workflow presented in operation 812 of the method 800. The graphical user interface 1000 allows the user to search for a patient in the EHR system 600, and to select the patient such that the examination recorded by the imaging device 400 is associated with the patient. This enables storage of the examination recorded by the imaging device 400 in the EHR 602 of the patient. Further, the metrics captured in operation 808 can include identification of the patient.

In the example provided in FIG. 10, the graphical user interface 1000 includes a first field 1002 for the user to enter a first name of the patient, and a second field 1004 for the user to enter a last name of the patient. The graphical user interface 1000 can include additional fields for entering additional information such as for entering a medical record number of the patient. Once one or more inputs are entered in the fields displayed on the graphical user interface 1000, the user can select a search icon 1006 to search for the patient within the EHR system 600.

The user can alternatively select a scan icon 1008 that causes display of a graphical user interface on the display 404 enabling the camera 410 to scan a machine-readable label such as a barcode or QR code to identify the patient in the workflow presented in operation 812.

The graphical user interfaces in the workflow presented in operation 812 are based on the type of optical viewing device 100 determined in operation 810. The graphical user interfaces are optimized for capturing images and/or videos of an anatomy that is typically examined by the type of optical viewing device 100 determined in operation 810 such as an eye anatomy examined by an ophthalmoscope, an ear anatomy examined by an otoscope, and a skin anatomy examined by a dermatoscope. The graphical user interfaces in the workflow presented in operation 812 can further include one or more tools for annotating a video of the anatomy captured by the imaging device 400, or one or more images or frames selected from the video.

For example, the workflow can include tools such as predefined annotations associated with a type of anatomy typically examined by the type of optical viewing device 100 determined in operation 810. The predefined annotations are selectable on the display 404 to indicate presence of a condition or disease state associated with the type of anatomy. The workflow can also include tools such as filters based on the type of anatomy typically examined by the type of optical viewing device 100 determined in operation 810. Filters such as a grayscale filter, a high-contrast filter, and a red-free filter can be presented for use on the imaging device 400 based on the type of optical viewing device 100 determined in operation 810. The workflow can also include tools such as a ruler superimposed on an image or frame selected from the video of the anatomy, and the ruler is scaled based on the type of anatomy typically examined by the type of optical viewing device 100 determined in operation 810. The ruler can provide a reference for one or more anatomical features included in the selected frame.

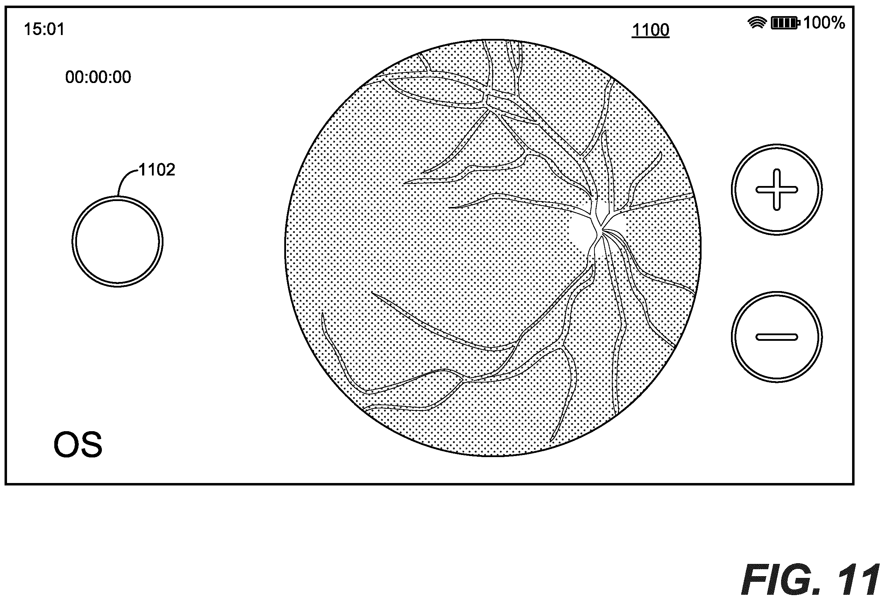

FIG. 11 illustrates an example of a graphical user interface 1100 that is optimized for capturing a video and/or images through the eyepiece 201 of an ophthalmoscope (e.g., the second type of optical viewing device 104). The graphical user interface 1100 includes a record icon 1102 that can be selected by the user to record the video of the eye anatomy. Additional types of graphical user interfaces can be displayed on the display 404 such as a graphical user interface that is optimized for capturing a video and/or images through the eyepiece 201 of an otoscope (e.g., the first type of optical viewing device 102), a graphical user interface that is optimized for capturing a video and/or images through the eyepiece 201 of a dermatoscope (e.g., the third type of optical viewing device 102), or of another type of optical viewing device.

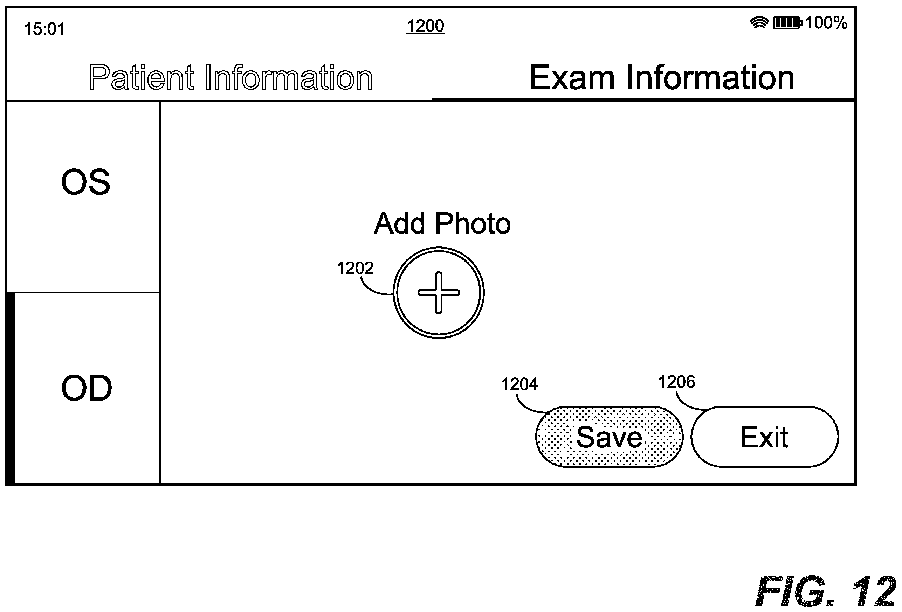

FIG. 12 illustrates an example of a graphical user interface 1200 that can be displayed in the workflow presented in operation 812. The graphical user interface 1200 is generated for use when the imaging device is attached to an ophthalmoscope (e.g., the second type of optical viewing device 104). The graphical user interface 1200 includes an icon 1202 that when selected allows the user to capture another image or video of the left eye (OS) or right eye (OD). The graphical user interface 1200 further includes a save icon 1204 to save the examination, and an exit icon 1206 to exit the examination and discard the captured images and/or videos.

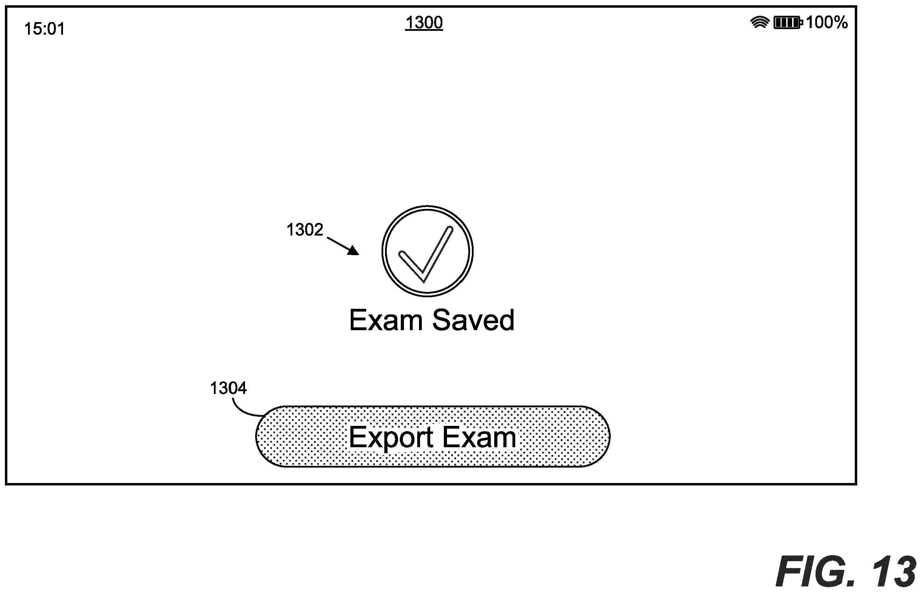

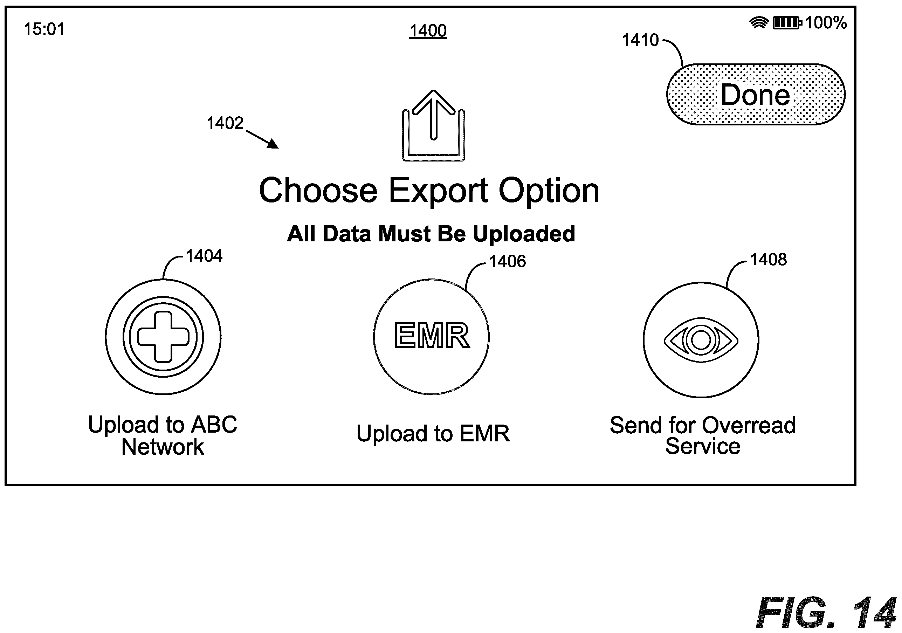

FIG. 13 illustrates an example of a graphical user interface 1300 that can be displayed in the workflow presented in operation 812 after the user selects the save icon 1204 in the graphical user interface 1200 of FIG. 12. The graphical user interface 1300 includes a confirmation 1302 that the recording of the examination by the imaging device 400 is saved. The graphical user interface 1300 further includes an export icon 1304 that when selected allows the user to export an examination report to another device or system such as the EHR system 600 or the overread system 700 (see FIG. 1). An export screen 1400 (see FIG. 14) is generated in response to selection of the export icon 1304, as will be described in more detail further below.

Referring back to FIG. 8, the method 800 includes an operation 814 of determining whether the examination that is being recorded by the imaging device 400 is complete. Operation 814 can include determining the examination is complete when the save icon 1204 is selected on the graphical user interface 1200. As another example, operation 814 can include determining the examination is complete when the imaging device 400 remains idle for more than a predetermined period of time. As another example, operation 814 can include determining the examination is complete when the optical viewing device 100 is moved away from an anatomy such as an ear, eye, or skin surface for a predetermined period of time such that the imaging device 400 is no longer capturing an image or video of the anatomy.

When operation 814 determines the examination is not complete (i.e., "No" in operation 814), the method 800 can continue to present the workflow (operation 812) and capture the metrics (operation 808). When operation 814 determines the examination is complete (i.e., "Yes" in operation 814), the method 800 proceeds to an operation 816 of presenting an export screen, which as described above, allows the user to export a report that includes the images, videos, and other data captured by the imaging device 400 during the examination to another device or system such as the EHR system 600 or the overread system 700 (see FIG. 1).

FIG. 14 illustrates an example of the export screen 1400 that is generated in operation 816 of the method 800. The export screen 1400 displays a warning 1402 that all data including the images, videos, and other data captured during the examination must be uploaded to another device or system such as the EHR system 600 or the overread system 700. As described above, the images, videos, and other data are deleted from the RAM 2010 of the imaging device 400 after the images, videos, and other data are uploaded to another device or system to maintain the confidentiality of protected health information captured on the imaging device 400.

The export screen 1400 includes a first export option 1404 selectable to export the images, videos, and other data to a secure network. For example, selection of the first export option 1404 causes the images, videos, and other data to be sent to a file hosting service such as Microsoft OneDrive^{®}, Google Driver, iCloud^{®}, and other similar file hosting services. Alternatively, selection of the first export option 1404 causes the images, videos, and other data to be sent to a proprietary network maintained by a manufacturer of the imaging device 400.

The export screen 1400 includes a second export option 1406 selectable to export the images, videos, and other data to the EHR 602 hosted on the EHR system 600. The images, videos, and other data captured by the imaging device 400 can be stored directly in the EHR of a patient after examination of the patient by using the optical viewing device 100 is complete.

The export screen 1400 includes a third export option 1408 that is selectable to export the images, videos, and other data to the overread system 700 (see FIG. 1). As described above, the overread system 700 can provide the images, videos, and other data captured by the imaging devices 400 for review by trained medical specialists or technicians for disease screening and diagnosis. Alternatively, or additionally, the overread system 700 can perform algorithms such as artificial intelligence and machine learning to analyze the images, videos, and data captured by the imaging devices 400 for disease screening and diagnosis.

The first export option 1404, the second export option 1406, and the third export option 1408 can be selected either individually or in combination with one another. For example, a user of the imaging device 400 can decide to upload the images, videos, and other data to both the EHR 602 of the patient, and to the overread system 700. When the user is satisfied with one or more selections of the first export option 1404, and/or the second export option 1406, and/or the third export option 1408, the user can select the done icon 1410 to enter the selection(s).

The export screen 1400 prevents insecure data transmission by limiting the export of the images, videos, and other data captured by the imaging device 400 to secure destinations such as a secure network, the EHR system 600, and the overread system 700. For example, the export screen 1400 prevents exporting the images, videos, and other data captured by the imaging device 400 to a universal serial bus (USB) drive, which can be stolen and accessed by an unauthorized user. Also, the export screen 1400 prevents exporting the images, videos, and other data captured by the imaging device 400 via e-mail or text message which are insecure data transfer methods. Instead, the export screen 1400 causes the images, videos, and other data captured by the imaging device 400 to be exported to secure destinations that require appropriate user credentials to retrieve and access the data such as username, password, PIN, and the like.

In some examples, instead of, or in addition to receiving inputs on the export screen 1400, the images, videos, and other data captured by the imaging device 400 are automatically sent to a secure destination when the imaging device 400 connects to a computing device that is recognized by the software installed on the imaging device 400. Such computing device can have installed thereon proprietary software developed by the manufacturer of the imaging device 400. Also, the images, videos, and other data captured by the imaging device 400 can be automatically sent to a secure destination when the imaging device 400 connects to a recognized network or cloud server. In such examples, the images, videos, and other data captured by the imaging device 400 can be automatically synchronized between the imaging device 400 and one or more secured destinations without requiring input on the export screen 1400.

In some examples, the imaging device 400 requests a wireless handshake to connect the imaging device 400 to another device. For example, the wireless handshake can include an exchange of one or more keys over Bluetooth^{®} or other wireless protocol to authenticate the other device. After the other device is authenticated, the imaging device 400 can transfer the images, videos, and other data to the other device over Wi-Fi or other suitable wireless network protocol. In some examples, the images, videos, and other data captured by the imaging device 400 are encrypted for communication over wired and/or wireless networks to further ensure confidentiality of the protected health information captured on the imaging device 400.

Referring back to FIG. 8, the method 800 includes an operation 818 of receiving export instructions. Operation 818 includes receiving the export instructions based on the selection(s) made on the export screen 1400 such as a selection of the first export option 1404 to upload the examination report to a secure network, and/or a selection of the second export option 1406 to upload the examination report to the EHR 602 of the patient, and/or a section of the third export option 1408 to upload the examination report to the overread system 700. Additionally, or alternatively, operation 818 can include receiving the export instructions when the imaging device 400 connects to a computing device, network, or cloud server that is recognized by the software installed on the imaging device 400.

The method 800 includes an operation 820 of generating an examination report based on the images, videos, and other data captured by the imaging device 400. As discussed above, the images, videos, and other data captured by the imaging device 400 can be of an ear anatomy when the imaging device 400 is attached to an otoscope (e.g., the first type of optical viewing device 102). Alternatively, the images, videos, and other data captured by the imaging device 400 can be of an eye anatomy when the imaging device 400 is attached to an ophthalmoscope (e.g., the second type of optical viewing device 104). Alternatively, the images, videos, and other data captured by the imaging device 400 can be of a skin anatomy when the imaging device 400 is attached to a dermatoscope (e.g., the third type of optical viewing device 106).

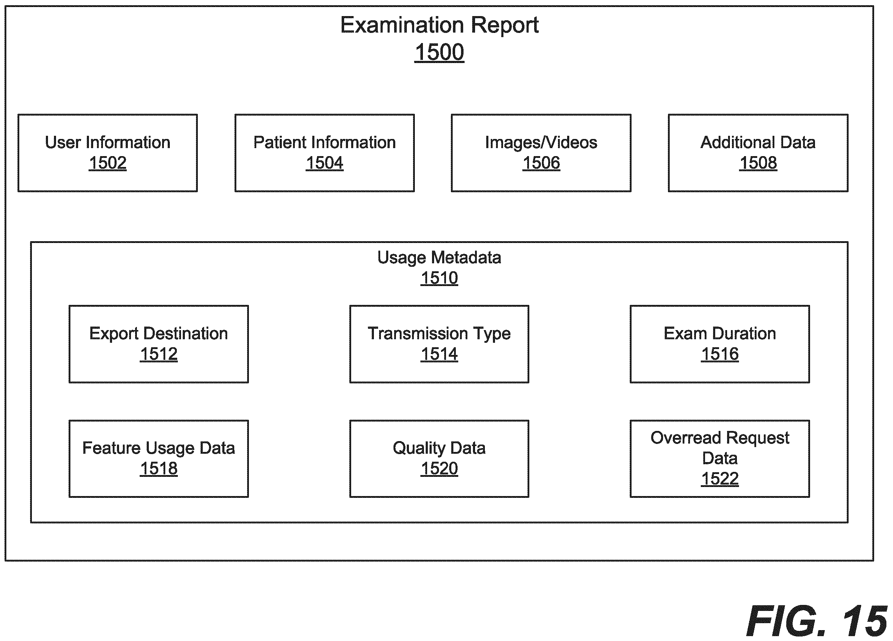

FIG. 15 schematically illustrates an example of an examination report 1500 generated in operation 820 of the method 800. The examination report 1500 includes user information 1502 such as the user's name, employment location (e.g., a healthcare facility such as a hospital or a medical clinic or office where the user is employed), title/role (e.g., physician, registered nurse, and the like), and specialty (e.g., whether the user is general practitioner or has a specialty such as an ear, nose, and throat (ENT) specialist, an ophthalmologist, or a dermatologist). The user information 1502 can be retrieved from a database located off the imaging device 400 based on the user credentials received in operation 804 (see also graphical user interface 900 of FIG. 9).

The examination report 1500 includes patient information 1504 such as patient name, date of birth, gender, and/or medical record number. The patient information 1504 can be retrieved from a database located off the imaging device 400 based on the patient selected from the search criteria entered in the graphical user interface 1000 of FIG. 10.

The examination report 1500 includes images and/or videos 1506 captured by the camera 410 of the imaging device 400. The images and/or videos 1506 are captured using the graphical user interfaces optimized for capturing the images and/or videos through the eyepiece 201 of the optical viewing device 100 such as the graphical user interface 1100 of FIG. 11.

The examination report 1500 includes additional data 1508 such as annotations, markings, notes, and the like that can be added by the user to the images and/or videos that are captured by the camera 410 of the imaging device 400. The additional data 1508 can also include preliminary diagnoses of diseases or conditions observed by the user. The additional data 1508 can be added by the using the tools included on the graphical user interfaces in the workflow presented on the display 404 of the imaging device in accordance with operation 812.

The examination report 1500 further includes usage metadata 1510 based on the metrics captured in operation 808. In alternative examples, the usage metadata 1510 can be attached to a video file or an image file that is sent from the imaging device 400 to the user analytics system 500, the EHR system 600, and/or the overread system 700.

As shown in FIG. 15, the usage metadata 1510 includes an export destination 1512 based on whether the user selected one or more secure destinations for exporting the images, videos, and other data captured by the imaging device 400 such as a secure network, an EHR 602 of the patient hosted on the EHR system 600, and/or the overread system 700.

The usage metadata 1510 can further include a transmission type 1514 such as whether the images, videos, and other data captured by the imaging device 400 are exported to the one or more secure destinations via a wireless connection via the network 2052, or through a wired connection to a computing device recognized by the imaging device 400.

The usage metadata 1510 can further include an exam duration 1516 recorded by the imaging device 400. For example, the metrics captured in operation 808 of the method 800 can include how long it took the user to complete the examination of the left and/or right ears using an otoscope (e.g., the first type of optical viewing device 102), or how long it took the user to complete the examination of the left and/or right eyes using an ophthalmoscope (e.g., the second type of optical viewing device 104), or how long it took the user to complete the examination of a skin surface using a dermatoscope (e.g., the third type of optical viewing device 106). By tracking the exam duration 1516, the efficiency of the user can be monitored.

The usage metadata 1510 can further include data related to the users interactions with the graphical user interfaces in the workflow presented in operation 812 of the method 800. For examples, the usage metadata 1510 can include feature usage data 1518 that identifies which features or tools were used by the user of the imaging device 400. The feature usage data 1518 is captured in operation 808 of the method 800. As discussed above, the features or tools can include predefined annotations, filters, rulers, and the like for annotating a video or an image of an anatomy seen through the eyepiece 201 of the optical viewing devices 100.

As an illustrative example, the feature usage data 1518 can identify which tools were used by the user of the imaging device 400 while examining the left and/or right ears using an otoscope (e.g., the first type of optical viewing device 102). As another example, the feature usage data 1518 can identify which tools were used by the user of the imaging device 400 while examining the left and/or right eyes using an ophthalmoscope (e.g., the second type of optical viewing device 104). As a further example, the feature usage data 1518 can identify which tools were used by the user of the imaging device 400 while examining a skin surface using a dermatoscope (e.g., the third type of optical viewing device 106).

The usage metadata 1510 can further include image quality data 1520 which characterizes the quality of the videos and/or images captured by the imaging device 400. The image quality data 1520 can include an overall quality score that is computed based on the video or images captured by the camera 410. The overall quality score can range in a scale such as from 0 to 100. The image quality data 1520 can further include individual quality characteristics such as lighting, contrast, sharpness, artifacts, noise, flare, color accuracy, distortion, and other image quality attributes. The image quality data 1520 can further include whether desired anatomical features are within the videos and/or images captured by the camera 410.

In some examples, the image quality data 1520 can be determined by one or more local algorithms that run on the imaging device 400. In such examples, the image quality data 1520 can be captured in operation 808 of the method 800 while the imaging device 400 is being used to capture the videos and/or images during an examination of a patient.

Alternatively, the image quality data 1520 can be determined by another system such as the overread system 700. For example, the overread system 700 can utilize one or more algorithms including artificial intelligence and/or machine learning to characterize the quality of the videos and/or images transmitted from the imaging device 400 to the overread system 700. Thereafter, the overread system 700 can transfer the image quality data 1520 to the user analytics system 500, or the imaging device 400 for inclusion in the examination report 1500.

The usage metadata 1510 can include overread request data 1522 such as whether one or more overread services or referrals are requested by the user of the imaging device 400. The overread request data 1522 can specify a type of overread service request such as whether review by a human technician, or review by an algorithm such as artificial intelligence.

Referring back to FIG. 8, the method 800 includes an operation 822 of sending the examination report 1500 to a secure destination such as a secure network, the EHR system 600, and/or the overread system 700. As described above, certain aspects are implemented to protect the confidentiality of protected health information that may be included in the examination report. For example, when the imaging device 400 is physically connected to another device to transfer of the examination report 1500, the imaging device 400 can require that the other device have installed thereon proprietary software developed by the manufacturer of the imaging device 400 such that the other device is recognizable by the imaging device 400. Further, the imaging device 400 can require a handshake to connect the imaging device 400 to a wireless network. Also, the images, videos, and other data captured by the imaging device 400 are encrypted for communication over wired and/or wireless networks to further ensure confidentiality of the protected health information that is captured and transferred by the imaging device 400.

The method 800 includes an operation 824 of deleting the images, the videos, and the other data from a memory of the imaging device 400 after the examination report 1500 is sent in operation 822. This ensures that protected health information does not remain on the imaging device 400, and thereby improves the confidentiality of the protected health information.

The method 800 further includes an operation 826 of displaying training on the display 404 of the imaging device. The training is based on the usage metadata 1510 included in the examination report 1500. The usage metadata 1510 can be used to identify one or more areas to improve user competency of the imaging device 400 and/or the optical viewing device 100. For example, the usage metadata 1510 can identify efficiency issues related to use of the imaging device 400 and/or the optical viewing device 100, image quality issues related to use of the imaging device 400 and/or the optical viewing device 100, and/or issues related to utilizing the tools on the imaging device 400 including usage of the overread system 700 that could improve the efficacy of the imaging device 400 and/or the optical viewing device 100.

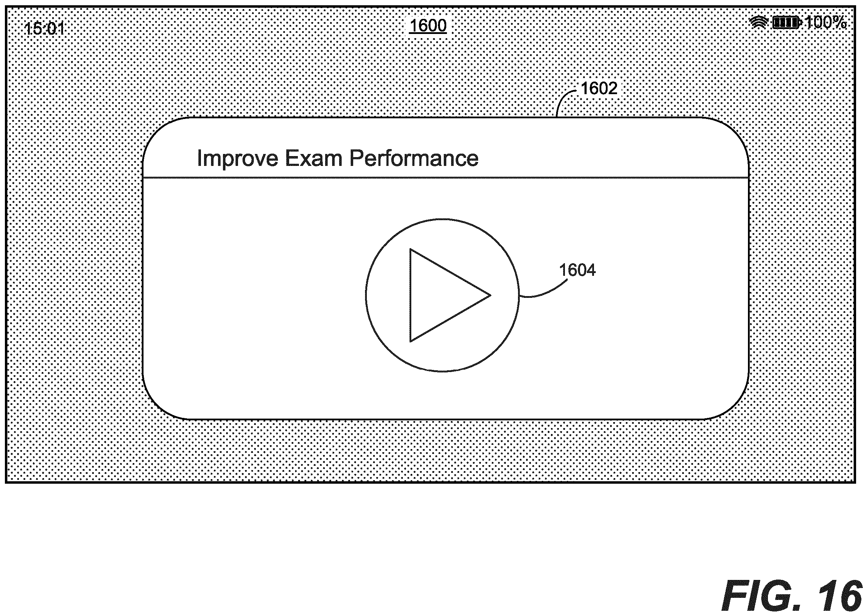

FIG. 16 illustrates an example of a graphical user interface 1600 that can be displayed in operation 826 of the method 800 after the examination report is sent (operation 822) and the images, the videos, and the other data are deleted from the memory of the imaging device 400 (operation 824). The graphical user interface 1600 includes a training window 1602 that includes a play icon 1604 that is selectable to playback a video that describes techniques to improve user competency of the imaging device 400 and/or the optical viewing device 100 based on the usage metadata 1510 collected by the imaging device 400.

As an example, when the exam duration 1516 in the usage metadata 1510 indicates that the user is taking longer than what is typically necessary to complete an examination of the left and/or right ears using an otoscope (e.g., the first type of optical viewing device 102), an examination of the left and/or right eyes using an ophthalmoscope (e.g., the second type of optical viewing device 104), or an examination of a skin surface using a dermatoscope (e.g., the third type of optical viewing device 106), the play icon 1604 when selected causes playback of a training video inside the training window 1602 that provides tips for using the imaging device 400 and/or the optical viewing device 100 more efficiently. For example, the tips on how to adjust one or more parameters such as lighting and/or zoom on the optical viewing device 100 that can make examination of a desired anatomy easier, thereby improving exam efficiency.

As another example, when the image quality data 1520 in the usage metadata 1510 indicates that the quality of the videos and images captured by the camera 410 of the imaging device 400 is poor, the play icon 1604 when selected causes playback of a training video inside the training window 1602 that provides tips to improve image quality. The training video can provide suggestions to adjust the lighting, or hold the optical viewing device 100 closer or farther away from an anatomical area of interest, or hold the optical viewing device 100 more steadily to mitigate blurred imaging that results from movements of the user's hand.

As another illustrative example, when the feature usage data 1518 included in the usage metadata 1510 indicates that the user did not utilize one or more tools to annotate the videos and images captured by the camera 410 of the imaging device 400, the play icon 1604 when selected causes playback of a training video inside the training window 1602 that provides a recommendation on how to use the one or more tools to improve exam efficacy.

As another illustrative example, when the overread request data 1522 in the usage metadata 1510 indicates that the user did not elect to export the images, videos, and other data to the overread system 700 (i.e., no selection of the third export option 1408 in the export screen 1400), the play icon 1604 when selected causes playback of a training video inside the training window 1602 that recommends use the overread system 700 to improve disease screening.

In some examples, the imaging device 400 presents a graphical user interface that enables the user to adjust one or more settings of the imaging device 400 based on the recommendations included in the training displayed in operation 826. In some further examples, the imaging device 400 automatically adjusts one or more settings of the imaging device 400 based on the recommendations included in the training displayed in operation 826.

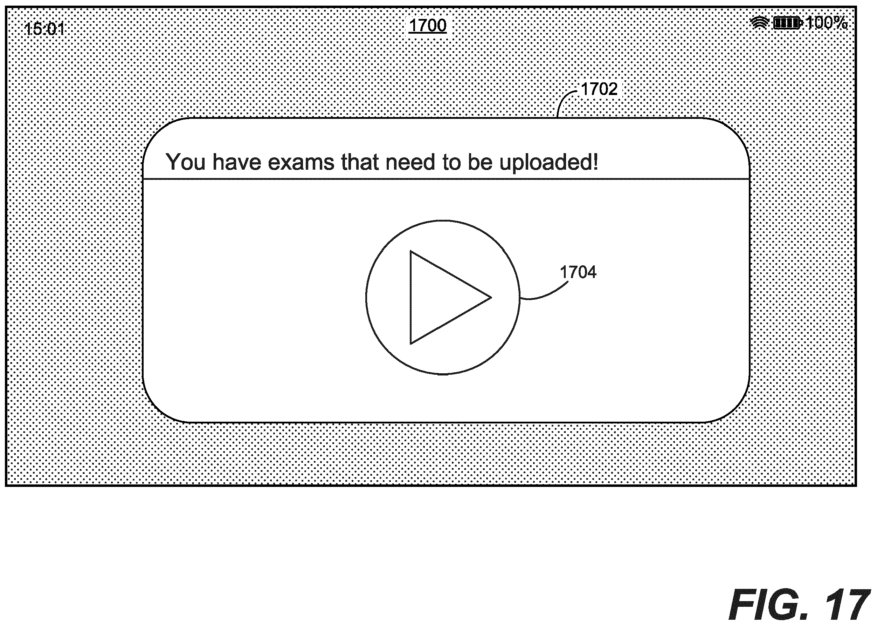

FIG. 17 illustrates another example of a graphical user interface 1700 that can be displayed in operation 826. Alternatively, the graphical user interface 1700 can be displayed after the user credentials are validated in operation 806 of the method 800. The graphical user interface 1700 includes a training window 1702 that includes a message that the user has exams the need to be uploaded to a secure destination such as a secure network, the EHR system 600, or the overread system 700. The training window 1702 further includes a play icon 1704 that is selectable to playback a video inside the training window 1702 that explains how to upload the exams to a secure destination such as by using the export screen 1400.

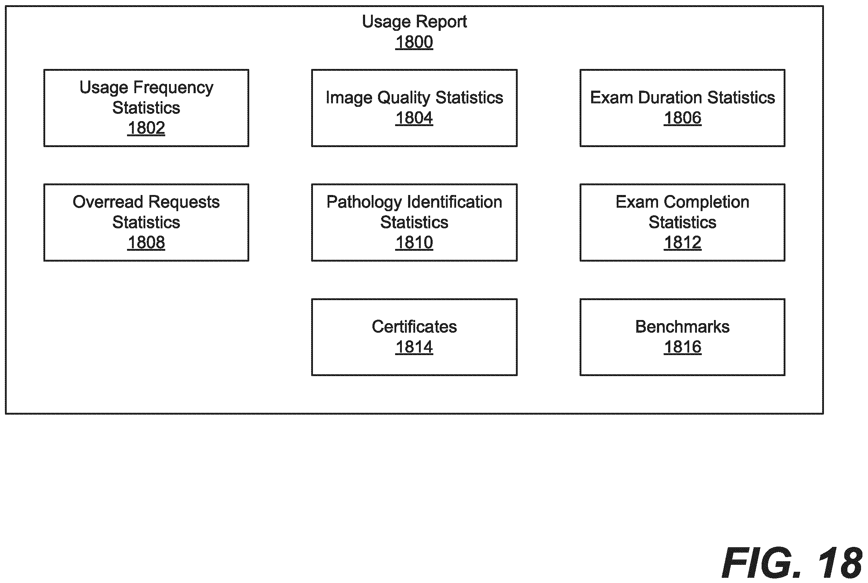

FIG. 18 schematically illustrates an example of a usage report 1800 that can be generated by the user analytics system 500. The usage report 1800 displays usage statistics for one or more imaging devices 400 that are used by a plurality of users within a healthcare facility such as a hospital, a medical clinic, medical office, and the like. The usage report 1800 is generated based on the usage metadata 1510 included in the examination reports 1500 that are generated by the one or more imaging devices 400. The usage report 1800 can further include recommendations to improve user competency of the one or more imaging devices 400 and/or the optical viewing devices 100. The usage report 1800 can be generated by the user analytics system 500 periodically such as weekly, monthly, or quarterly.

The usage report 1800 includes usage frequency statistics 1802, image quality statistics 1804, exam duration statistics 1806, overread requests statistics 1808, pathology identification statistics 1810, and exam completion statistics 1812, which can be calculated per individual user, or per user population such as doctors, registered nurses, medical residents, trainees, students, and other user populations within the medical facility.

The usage frequency statistics 1802 include data on how frequently the imaging devices 400 were used per individual user, or per user population. For example, the usage frequency statistics 1802 can identify that the imaging devices 400 were used more frequently by nurse practitioners or other allied health providers than by doctors. The usage frequency statistics 1802 can further identify which particular users used the imaging devices 400 most frequently, and which particular users used the imaging devices 400 least frequently. The usage frequency statistics 1802 can be used to identify a particular user population or an individual user who should be targeted for additional training to increase their use of the imaging devices 400 on the optical viewing devices 100 to improve patient screenings within the medical facility.

The image quality statistics 1804 include data on the quality of the videos and images captured using the imaging devices 400 per individual user, or per user population. For example, the image quality statistics 1804 can identify which users captured the best quality videos and images, and which users captured the videos and images having the poorest quality. The image quality statistics 1804 can be used to identify a particular user population or an individual user who should be targeted for additional training to improve their competency of the imaging devices 400 such as how to effectively use the devices to improve video and image quality.

The exam duration statistics 1806 include data on exam duration per individual user, or per user population. For example, the exam duration statistics 1806 can identify which users took the longest time to complete an examination of the left and/or right ears using an otoscope (i.e., the first type of optical viewing device 102), which users took the longest time to complete an examination of the left and/or right eyes using an ophthalmoscope (i.e., the second type of optical viewing device 104), or which users took the longest time to complete an examination of a skin surface using a dermatoscope (i.e., the third type of optical viewing device 106). The exam duration statistics 1806 can be used to identify a particular user population or an individual user who should be targeted for additional training to improve their efficiency by reducing exam duration when using the imaging devices 400 on the optical viewing devices 100.

The overread requests statistics 1808 include data on the quantity of overread requests sent using the imaging devices 400 per individual user, or per user population. For example, the overread requests statistics 1808 can identify which user population or individual user sent the most amount of overread requests, and which user population or individual user sent the least amount of overread requests. The overread requests statistics 1808 can be used to identify a particular user population or an individual user who should be targeted for additional training to improve disease screening by increasing overread requests sent by the imaging devices 400.

The pathology identification statistics 1810 include data on quantities of diseases, medical conditions, and pathologies identified per individual user, or per user population. For example, the pathology identification statistics 1810 can quantify which diseases, medical conditions, and pathologies were identified per user population or individual user based on the videos and images captured by the imaging devices 400. The pathology identification statistics 1810 can further identify which user population or individual user had the highest false positive rate and highest false negative rate based on confirmation of the diseases, medical conditions, and pathologies by the overread system 700. The pathology identification statistics 1810 can be used to identify a particular user population or an individual user who should be targeted for additional training to reduce false positive rates and reduce false negative rates.

The exam completion statistics 1812 include data on a quantity of incomplete exams per individual user, or per user population. For example, the exam completion statistics 1812 can identify which user population or individual user has the largest quantity of incomplete exams. The exam completion statistics 1812 can be used to identify a particular user population or an individual user who should be targeted for additional training to reduce the quantities of incomplete exams stored on the imaging devices 400.

The user analytics system 500 can generate certificates 1814 to reward individual users of the imaging devices 400 and/or user populations within the medical facility. The certificate 1814 are generated by the user analytics system 500 based on the usage frequency statistics 1802, the image quality statistics 1804, the exam duration statistics 1806, the overread requests statistics 1808, the pathology identification statistics 1810, and exam completion statistics 1812. As an illustrative example, the user analytics system 500 can generate a certificate 1814 to reward an individual user and/or user population who uses the imaging device 400 most frequently based on the usage frequency statistics 1802.

As another example, the user analytics system 500 can generate a certificate 1814 to reward an individual user and/or user population who captures the highest quality videos and images using the imaging device 400 based on the image quality statistics 1804.

As a further example, the user analytics system 500 can generate a certificate 1814 to reward an individual user and/or user population who has a lowest false positive rate and/or a lowest false negative rate based on the pathology identification statistics 1810.

As another example, the user analytics system 500 can generate a certificate 1814 to reward an individual user and/or user population who has the fewest number of incomplete exams based on the exam completion statistics 1812.

Further, the user analytics system 500 can generate benchmarks 1816 for comparing a medical facility with a benchmark medical facility that shares one or more common characteristics such as geographical location, size (e.g., patient capacity, number of trained medical staff, square footage, etc.), and/or medical specialty (e.g., whether general practice, or specialized practice such as ear, nose, and throat (ENT), ophthalmology, dermatology, etc.).

The benchmarks 1816 are generated by the user analytics system 500 based on the usage frequency statistics 1802, the image quality statistics 1804, the exam duration statistics 1806, the overread requests statistics 1808, the pathology identification statistics 1810, and the exam completion statistics 1812. The benchmarks 1816 can be used to display one or more statistical comparisons such as to indicate whether the imaging devices 400 are used more frequently or less frequently in the medical facility than in the benchmark medical facility.

In a further example, the benchmarks 1816 can be used to display a comparison of one or more average quality characteristics of the images and videos captured by the imaging devices 400 in the medical facility with one or more average quality characteristics of the images and videos captured by imaging devices 400 in the benchmark medical facility.

As another example, the benchmarks 1816 can be used to display a comparison of the average exam duration captured by the imaging devices 400 in the medical facility with the average exam duration captured by imaging devices 400 in the benchmark medical facility.

Also, the benchmarks 1816 can be used to display a comparison of a quantity of overread requests sent from the imaging devices 400 in the medical facility with a quantity of overread requests sent from the imaging devices 400 in the benchmark medical facility.

Additionally, the benchmarks 1816 can be used to display a comparison of a quantity of pathologies identified using the imaging devices 400 in the medical facility with a quantity of pathologies identified using the imaging devices 400 in the benchmark medical facility.

Also, the benchmarks 1816 can be used to display a comparison of a quantity of exams that remain incomplete on the imaging devices 400 in the medical facility with a quantity of exams that remain incomplete on the imaging devices 400 in the benchmark medical facility.

The benchmarks 1816 can also be used to display comparisons between one or more different time periods or ranges (e.g., weeks, months, quarters, and the like). For example, the benchmarks 1816 can be used to compare usage frequency, image quality statistics, average exam duration, overread request statistics, pathology identification statistics, and exam completion statistics for the imaging devices 400 within the medical facility during a first time period with a second time period such that a user or administrator in the medical facility can visually view competency progress in using the imaging devices 400 and/or the optical viewing devices 100 based on the training provided on the graphical user interfaces 1600, 1700.

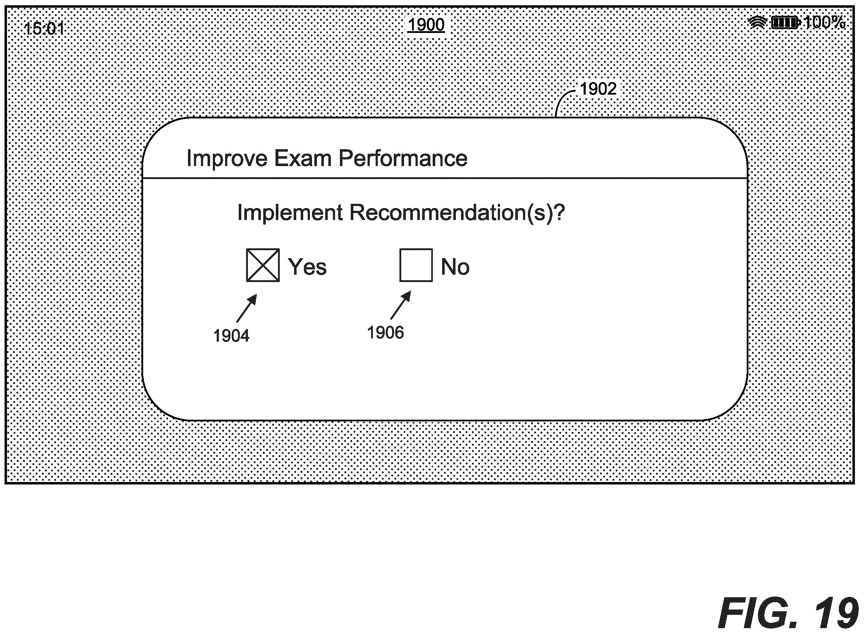

FIG. 19 illustrates an example of a graphical user interface 1900 that can be displayed on the imaging device 400. The graphical user interface 1900 includes a training window 1902 that is displayed after playback of a video that describes techniques to improve user competency of the imaging device 400 and/or the optical viewing device 100 based on the usage metadata 1510 collected by the imaging device 400 (e.g., see graphical user interface 1600 of FIG. 16). Alternatively, the training window 1902 is displayed after playback of a video that explains how to upload the exams to a secure destination (e.g., see graphical user interface 1700 of FIG. 17).

In FIG. 19, the training window 1902 includes a first input option 1904 that when selected causes one or more recommendations that are suggested in the video to be implemented on the imaging device 400. The training window 1902 also includes a second input option 1906 that when selected declines to implement the recommendations that are suggested in the video.

As an illustrative example, the first input option 1904 when selected causes one or more settings such as illumination, zoom, and the like to be adjusted on the imaging device 400 to improve quality of the images and/or videos captured by the camera 410, improve efficiency by shortening exam duration, and/or improve user competency of the imaging device 400 and/or the optical viewing devices 100. As another illustrative example, the first input option 1904 when selected causes one or more features to switch from disabled to enabled to take full advantages of the features of the imaging device 400 to enhance disease screening.

FIG. 20 illustrates an exemplary architecture of a computing device 2000 of the imaging device 400. The computing device 2000 is used to execute the functionality of the imaging device 400 described herein. The imaging device 400 can include all or some of the elements described with reference to FIG. 20, with or without additional elements.

The computing device 2000 includes at least one processing device 2002. Examples of the at least one processing device 2002 can include central processing units (CPUs), digital signal processors, field-programmable gate arrays, and other types of electronic computing circuits. The at least one processing device 2002 can be part of a processing circuitry having a memory for storing instructions which, when executed by the processing circuitry, cause the processing circuitry to perform the functionalities described herein.

The computing device 2000 also includes a system memory 2004, and a system bus 2006 that couples various system components including the system memory 2004 to the at least one processing device 2002. The system bus 2006 can include any type of bus structure including a memory bus, or memory controller, a peripheral bus, and a local bus.

The system memory 2004 may include a read only memory (ROM) 2008 and a random-access memory (RAM) 2010. An input/output system containing routines to transfer information within the computing device 2000, such as during start up, can be stored in the read only memory (ROM) 2008. The system memory 2004 can be housed inside the housing 402.

The computing device 2000 can further include a secondary storage device 2014 for storing digital data. The secondary storage device 2014 is connected to the system bus 2006 by a secondary storage interface 2016. The secondary storage devices and computer-readable media provide nonvolatile storage of computer-readable instructions including application programs and program devices, data structures, and other data.

A number of program devices can be stored in secondary storage device 2014 or the system memory 2004, including an operating system 2018, one or more application programs 2020, other program modules 2022, and program data 2024. The system memory 2004 and the secondary storage device 2014 are examples of computer-readable data storage devices.

The computing device 2000 can include one or more input devices such as the display 404 (in examples where the display 404 is a touch sensitive touchscreen), one or more physical push buttons on the housing 402 of the imaging device 400, and the camera 410. Additional examples of input devices include a microphone 2026, and an accelerometer 2028 for image orientation on the display 404. The computing device 2000 can also include output devices such as the display 404, and a speaker 2030.

The input and output devices are connected to the at least one processing device 2002 through an input/output interface 2038 coupled to the system bus 2006. The input and output devices can be connected by any number of input/output interfaces, such as a parallel port, serial port, game port, or a universal serial bus. Wireless communication between the input and output devices and the input/output interface 2038 is possible as well, and can include Wi-Fi, Bluetooth, infrared, 802.1 1a/b/g/n, cellular, or other wireless communications.

In some examples, the display 404 is touch sensitive and is connected to the system bus 2006 via an interface, such as a video adapter 2042. The display 404 includes touch sensors for receiving input from a user when the user touches the display. Such sensors can be capacitive sensors, pressure sensors, or other touch sensors. The sensors detect contact with the display, and also the location and movement of the contact over time. For example, a user can move a finger or stylus across the display 404 to provide inputs.

The computing device 2000 further includes a communication device 2046 configured to establish communication across a network 2052. In some examples, when used in a local area networking environment or a wide area networking environment (such as the Internet), the computing device 2000 is typically connected to the network 2052 through a network interface, such as a wireless network interface 2050. The wireless network interface 2050 can provide Wi-Fi functionality such as for image and video transferring, live streaming, and providing a mobile hotspot. In some further examples, the wireless network interface 2050 can provide Bluetooth connectivity. Other possible examples using other wired and/or wireless communications are possible. For example, the computing device 2000 can include an Ethernet network interface, or a modem for communicating across the network.

In further examples, the communication device 2046 provides short-range wireless communication. The short-range wireless communication can include one-way or two-way short-range to medium-range wireless communication. Short-range wireless communication can be established according to various technologies and protocols. Examples of short-range wireless communication include a radio frequency identification (RFID), a near field communication (NFC), a Bluetooth technology, a Wi-Fi technology, or similar wireless technologies.

The computing device 2000 typically includes at least some form of computer-readable media. Computer-readable media includes any available media that can be accessed by the computing device 2000. By way of example, computer-readable media can include computer-readable storage media and computer-readable communication media.

Computer-readable storage media includes volatile and nonvolatile, removable, and non-removable media implemented in any device configured to store information such as computer-readable instructions, data structures, program devices, or other data. Computer-readable storage media includes, but is not limited to, random access memory, read only memory, electrically erasable programmable read only memory, flash memory or other memory technology, or any other medium that can be used to store the desired information and that can be accessed by the computing device 2000.

Computer-readable communication media embodies computer-readable instructions, data structures, program devices or other data in a modulated data signal such as a carrier wave or other transport mechanism and includes any information delivery media. Modulated data signal refers to a signal having one or more of its characteristics set or changed in such a manner as to encode information in the signal. By way of example, computer-readable communication media includes wired media such as a wired network or direct-wired connection, and wireless media such as acoustic, radio frequency, infrared, and other wireless media. Combinations of any of the above are also included within the scope of computer-readable media.

The computing device 2000 is an example of programmable electronics, which may include one or more computing devices, and when multiple computing devices are included, such computing devices can be coupled together with a suitable data communication network so as to collectively perform the various functions, methods, or operations disclosed herein.

The computing device 2000 can include a location identification device 2048. The location identification device 2048 is configured to identify the geolocation of the computing device 2000. The location identification device 2048 can use various types of geolocating or positioning systems, such as network-based systems, handset-based systems, SIM-based systems, Wi-Fi positioning systems, and hybrid positioning systems. Network-based systems utilize service provider's network infrastructure, such as cell tower triangulation. Handset-based systems typically use the Global Positioning System (GPS). Wi-Fi positioning systems can be used when GPS is inadequate due to various causes including multipath and signal blockage indoors. Hybrid positioning systems use a combination of network-based and handset-based technologies for location determination, such as Assisted GPS.

Embodiments of the invention can be described with reference to the following numbered clauses, with preferred features laid out in the dependent clauses:
1. A method of capturing images through an eyepiece of an optical viewing device, the method comprising:
   providing an imaging device for attachment to the optical viewing device;
   presenting a workflow on the imaging device for capturing the images based on a type of optical viewing device, the workflow including tools for annotating the images on a display;
   capturing metrics related to user interactions with the workflow, and quality characteristics of the images captured during the workflow; and
   generating a recommendation based on the metrics.
2. The method of clause 1, wherein the recommendation is to upload the images to at least one of a secure network, an electronic health record system, and an overread system.
3. The method of clause 2, further comprising:
   deleting the images after the images are uploaded to the at least one of the secure network, the electronic health record system, and the overread system.
4. The method of clause 1, wherein the recommendation is to adjust one or more settings on the imaging device based on the quality characteristics of the images.
5. The method of any of any preceding clause, further comprising:
   providing a video that is selectable for playback on the display, the video describing the recommendation to improve competency of the imaging device.
6. The method of any preceding clause, further comprising:
   providing an input on the display that when selected causes the recommendation to be implemented on the imaging device.

## Claims

1. An imaging device for capturing images through an eyepiece of an optical viewing device, the imaging device comprising:
a camera configured for alignment with the eyepiece of the optical viewing device for capturing the images viewed through the eyepiece;
a display for displaying the images captured by the camera;
at least one processing device; and
at least one computer readable data storage device storing software instructions that, when executed by the at least one processing device, cause the imaging device to:
determine a type of the optical viewing device;
present a workflow on the display based on the type of the optical viewing device; and
capture metrics related to user interactions with the workflow presented on the display and the images captured by the camera during the workflow.

2. The imaging device of claim 1, wherein the instructions, when executed by the at least one processing device, further cause the imaging device to:
generate a recommendation based on the metrics.

3. The imaging device of claim 2, wherein the recommendation is to upload the images to at least one of a secure network, an electronic health record system, and an overread system.

4. The imaging device of claim 3, wherein the instructions, when executed by the at least one processing device, further cause the imaging device to:
delete the images after the images are uploaded to the at least one of the secure network, the electronic health record system, and the overread system.

5. The imaging device of claim 2, wherein the recommendation is to adjust one or more settings on the imaging device.

6. The imaging device of any of claims 2-5, wherein the instructions, when executed by the at least one processing device, further cause the imaging device to:
provide a video on the display that is selectable for playback, the video describing the recommendation to improve competency of the imaging device.

7. The imaging device of any of claims 2-6, wherein the instructions, when executed by the at least one processing device, further cause the imaging device to:
provide an input on the display that when selected causes the recommendation to be implemented on the imaging device.

8. A method of capturing images through an eyepiece of an optical viewing device, the method comprising:
providing an imaging device for attachment to the optical viewing device;
presenting a workflow on the imaging device for capturing the images based on a type of optical viewing device, the workflow including tools for annotating the images on a display;
capturing metrics related to user interactions with the workflow, and quality characteristics of the images captured during the workflow; and
generating a recommendation based on the metrics.

9. The method of claim 8, wherein the recommendation is to upload the images to at least one of a secure network, an electronic health record system, and an overread system.

10. The method of claim 9, further comprising:
deleting the images after the images are uploaded to the at least one of the secure network, the electronic health record system, and the overread system.

11. The method of claim 8, wherein the recommendation is to adjust one or more settings on the imaging device based on the quality characteristics of the images.

12. The method of any of claims 8-11, further comprising:
providing a video that is selectable for playback on the display, the video describing the recommendation to improve competency of the imaging device.

13. The method of any of claims 8-12, further comprising:
providing an input on the display that when selected causes the recommendation to be implemented on the imaging device.

14. A system for imaging an anatomy, the system comprising:
an optical viewing device including an instrument head having an eyepiece; and
an imaging device as claimed in any one of claims 1 to 7.

15. The system of claim 14, wherein the optical viewing device is an otoscope, an ophthalmoscope, or a dermatoscope.
